(19) Europäisches Patentamt
European Patent Office
Office européen des brevets

(11) Publication number: 0 193 164
A2

(12) EUROPEAN PATENT APPLICATION

(21) Application number: 86102422.2

(22) Date of filing: 25.02.86

(51) Int. Cl.⁴: A 61 K 31/44
A 61 K 9/52, A 61 K 9/18

(30) Priority: 26.02.85 JP 36838/85

(43) Date of publication of application:
03.09.86 Bulletin 86/36

(84) Designated Contracting States:
AT BE CH DE FR GB IT LI LU NL SE

(71) Applicant: Fujisawa Pharmaceutical Co., Ltd.
3, Doshomachi 4-chome Higashi-ku
Osaka-shi, Osaka 541(JP)

(72) Inventor: Ueda, Yoshio
No. 1-3-5-204, Mikagenakamachi
Higashinada-ku Kobe(JP)

(72) Inventor: Furuyama, Setuo
No. 3-2-3, 907 Taishibashi
Asahi-ku Osaka(JP)

(72) Inventor: Shimojo, Fumio
No. 2-2-13, Daiwahigashi
Kawanishi(JP)

(72) Inventor: Shimazaki, Yasuo
No. 3-3-2-306, Akuraminami
Takarazuka(JP)

(74) Representative: Türk, Dietmar, Dr. rer. nat. et al,
Patentanwälte Türk & Gille Brucknerstrasse 20
D-4000 Düsseldorf 13(DE)

(54) **Sustained-release pharmaceutical preparation which comprised 2-nitroxy-methyl-6-chloropyridine, process for its preparation and use thereof.**

(57) A sustained-release pharmaceutical preparation which comprises 2-nitroxymethyl-6-chloropyridine or an occlusion compound thereof with β-cycloextrin, process for preparing it and use thereof as a medicament, particularly in the treatment of vascular disorder.

EP 0 193 164 A2

TS5376
0193164

SUSTAINED-RELEASE PHARMACEUTICAL PREPARATION WHICH COMPRISED 2-NITROXYMETHYL-6-CHLOROPYRIDINE, PROCESS FOR ITS PREPARTION AND USE THEREOF

The present invention relates to a sustained-release preparation for 2-nitroxymethyl-6-chloropyridine, which is represented by the following chemical formula ;

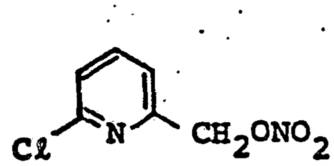

or its occlusion compound with β-cyclodextrin.

More particularly, the present invention relates to a sustained-release microcapsule aimed at keeping a constant level of the blood concentration of the active principal 2-nitroxymethyl-6-chloropyridine in the body for a long time.

2-Nitroxymethyl-6-chloropyridine has vasodilating activity and is useful as a drug for the treatment of

vascular disorder.

2-Nitroxymethyl-6-chloropyridine is, however, an oily and volatile substance, and therefore it is in general difficult to make up such substance into a pharmaceutical preparation.

Such difficulty could be overcome by converting 2-nitroxymethyl-6-chloropyridine to its occlusion compound with ß-cyclodextrin.

Generally, with regard to nitrate compounds, which are represented by nitroglycerin and also include 2-nitroxymethyl-6-chloropyridine, it is well known that after their oral administration, the rate of their absorption into blood and disappearance therefrom are very fast, and that therefore such compounds have the disadvantage of the short effective time.

After an inventive study, the inventors of the present invention have found that the above difficulty can be overcome by coating 2-nitroxymethyl-6-chloropyridine or its occlusion compound with ß-cyclodextrin, with released-control membrane to prepare a sustained-release microcapsule.

According to this microcapsule, the drug is released sustainedly and consequently and therefore is pharmacologically active for a long time in the body.

The present preparation has such an advantage; the release rate of the drug is nearly constant (zero order release) and further, is scarcely influenced by the change of the stirring intensity, pH of an outer medium and so on.

The present invention is described in more detail below.

The sustained-release microcapsule referred to the present invention is a microcapsule coated with semipermeable membrane, which gives the zero order release of the drug without the influence of the stirring condition

and pH of an outer medium in its dissolution behavior.

The sustained-release microcapsule is prepared by coating a granule containing the drug with a basic lubricant membrane.

The granules containing the drug is prepared by mixing the drug, excipient (e.g. cane sugar, lactose, sucrose, mannite, etc.) and other additives commonly used in this field in a conventional manner.

The amount of the drug is preferably 10 to 70 weight percent of the whole granule components, more preferably 40 to 60 weight percent of the whole granule components, but the amount of the drug is not restricted to the above range and may be determined fittingly depending on the intended duration of the drug release, or the like.

In these procedures, 2-nitroxymethyl-6-chloropyridine, the active principal, is such an oily and volatile substance that, this granule may be preferably prepared after conversion of 2-nitroxymethyl-6-chloropyridine to its occlusion compound with ß-cyclodextrin.

Then,the present invention refers to a method for releasing the drug sustainedly from such bed granules.

That is, such bed granules are coated with a basic libricant membrane which can control the release rate of the drug.

The basic lubricant membrane used here should be independent of pH of an outer medium, and may include a coating material such as acrylate resin [e.g. dimethylaminoethylmethacrylate - methylmetacrylic acid copolymer (e.g. Eudragit E 30 D etc.)], shellac, cellulose derivatives [e.g. ethylcellulose, hydroxypropyl-methylcellulose, etc.], or the like.

The amount of the coating material is preferably 0.1 to 20 weight percent of the whole bed granule containing the drug, but the amount is not restricted to the above range

and should be selected depending on the intended duration of drug release, or the like.

The coating treatment may be carried out with a fluid bed granulator in a conventional manner.

However, the method of the coating treatment is not restricted to the above procedure.

The detail of the present invention is described below using actual examples of producing preparation and evaluating their quality.

Preparation 1

Fuming nitric acid (1.57 ml) was added dropwise to acetic anhydride (3.54 ml) with stirring at 0° to 5°C. 2-Hydroxymethyl-6-chloropyridine (3.59 g) was added thereto. The resulting mixture was stirred for 20 minutes at 0° to 5°C. The resulting mixture was alkalized weakly with an aqueous potassium carbonate solution, extracted with chloroform, washed with water, dried over anhydrous magnesium sulfate, and concentrated under reduced pressure. The residue obtained was subjected to column chromatography on aluminum oxide (40 g) and eluted with toluene. The fractions containing the desired compound were concentrated under reduced pressure to give colorless viscous oil of 2-nitroxymethyl-6-chloropyridine (2.9 g).

IR (Nujol) : 1636, 1585, 1563, 1439, 1281, 1160, 1138, 985, 846, 785 $cm^{-1}$

NMR ($CCl_4$, δ) : 5.47 (2H, s), 7.15-7.45 (2H, m), 7.69 (1H, dd, J=8.5Hz, J=6Hz)

Analysis for $C_6H_5ClN_2O_3$

calcd C: 38.22 H: 2.67 N: 14.86

found C: 37.97 H: 2.75 N: 14.95

Preparation 2

In each of five centrifuge tubes, 600 mg of β-cyclodextrin was dissolved in 20 ml of distilled water. Following addition of 50, 100, 200, 300, and 400 mg of 2-nitroxymethyl-6-chloropyridine to the five centrifuge tubes, respectively, the mixtures were shaked, centrifuged and allowed to stand overnight at room temperature so as to complete crystallization.

The crystals were collected by filtration, washed with water and air-dried to give an occlusion compound of 2-nitroxymethyl-6-chloropyridine with β-cyclodextrin.

The conditions of preparing the occlusion compound of 2-nitroxymethyl-6-chloropyridine with β-cyclodextrin and the molar ratios found in the products are shown in Table 1.

The occlusion compound obtained has the following physical properties.

(i) In its X ray powder diffraction pattern, it shows peaks at about 11.5°, about 17.2° and about 18.4° which are characteristics of an occlusion compound of 2-nitroxymethyl-6-chloropyridine with β-cyclodextrin.

(ii) In its infrared absorption spectrum (Nujol), it shows absorptions at 3300, 1645, 1586, 1568, 1459, 1376, 1330, 1281, 1158, 1080, 1022, 1000, 940 and 842 $cm^{-1}$;

(iii) Sparingly soluble in water.

Table 1

Conditions of preparing an occlusion compound and molar ratios

| Composition of mixture solution (in 20 ml of distilled water) | | Content of 2-nitroxymethyl-6-chloropyridine in occlusion compound as obtained (%) | β-Cyclodextrin/2-nitroxymethyl-6-chloropyridine molar ratio in occlusion compound as obtained |
|---|---|---|---|
| Quantity of β-cyclodextrin added (mg) | Quantity of 2-nitroxymethyl-6-chloropyridine (mg) | | |
| 600 | 50 | 15.3 | 1/1.1 |
| | 100 | 14.5 | 1/1.0 |
| | 200 | 16.8 | 1/1.2 |
| | 300 | 34.2 | 1/3.1 |
| | 400 | 34.2 | 1/3.1 |

Example 1

Preparation of granule

(1) To Non-pariel (Trademark: prepared by Freund Industrial Company, 32 - 24 mesh) (1.0 kg) was added the occlusion compound of 2-nitroxymethyl-6-chloropyridine with ß-cyclodextrin (molar ratio 1:1) (1.3kg) which was already pulverized by jetmizer after the occlusion process.

The mixture was, then, powder-coated using 50% sucrose solution as a binder with a fluid bed granulator (CF 360) to form bed granules (2630 g, yield 98.9%).

(2) To a mixture of the occlusion compound of 2-nitroxymethyl-6-chloropyridine with ß-cyclodextrin (molar ratio 1:1) (160 g), Avicel PH 101 (Trademark: prepared by Asahi Chemical Industry Co., Ltd.) (12 g) and mannite (24 g) was added 5% aqueous hydroxypropylcellulose solution (30 ml) as a binder.

The mixture was subjected to kneading with a planetary mixer, to granulating with cylinder type granulator, to sphering with a marumerizer, and to drying to prepare granules (150g, yield 75%).

(3) To a mixture of the occlusion compound of 2-nitroxymethyl-6-chloropyridine with ß-cyclodextrin (molar ratio 1:1) (160 g), Avicel PH101 (12 g) and mannite (16 g) was added Eudragit E 30 D (Trademark: prepared by Rhom Pharma Darmstadt) (40 g) (12g as solid) an water (30 g) as a binder.

The mixture was subjected to kneading with a planetary mixer, to granulating with a cylinder type granulator, to sphering with a marumerizer, and to drying to prepare granules (50g, yield 75%).

Examples 2

prescription of coating solution

Several kinds of coating solutions which compositions are listed below were prepared in a conventional manner.

Eudragit formulation

| | | | |
|---|---|---|---|
| (1) | Eudragit E30D | 89.0 g | (26.9 g as solid) |
| | Talc | 6.6 g | |
| | Polyethylene glycol 6000 | 1.8 g | |
| | Distilled water | 171.0 g | |
| | | 268.4 g | (35.3 g as solid) |
| (2) | Eudragit E30D | 32.3 g | (9.7 g as solid) |
| | Talc | 2.4 g | |
| | Polyethylene glycol 6000 | 0.7 g | |
| | Distilled water | 62.0 g | |
| | | 97.4 g | (12.8 g as solid) |
| (3) | Eudragit RL 100 | 0.79 g | |
| | Eudragit RS 100 | 7.14 g | |
| | Talc | 1.59 g | |
| | Polyethylene glycol 6000 | 0.48 g | |
| | Ethanol | 44.20 g | |
| | Dichloromethane | 45.00 g | |
| | Fluorescein Sodium solution * | 0.80 g | |
| | | 100.00 g | (10.00 g as solid) |

Ethylcellulose formulation

| | | | |
|---|---|---|---|
| (4) | Ethylcellulose (10 cps) | 26.1 g | |
| | Talc | 6.6 g | |
| | Polyethylene glycol 6000 | 2.6 g | |
| | Ethanol | 318.0 g | |
| | | 353.3 g | (35.3 g as solid) |

| | | | |
|---|---|---|---|
| (5) | Ethylcellulose (10 cps) | 10.0 g | |
| | Polyethylene glycol 6000 | 10.0 g | |
| | Ethanol | 488.4 g | |
| | Dichloromethane | 490.0 g | |
| | Fluorescein Sodium solution * | 1.6 g | |
| | | 1000.0 g | (20.0 g as solid) |

| | | | |
|---|---|---|---|
| (6) | Ethylcellulose (10 cps) | 7.0 g | |
| | Polyethylene glycol 6000 | 3.0 g | |
| | Ethanol | 94.2 g | |
| | Dichloromethane | 95.0 g | |
| | Fluorescein Sodium solution * | 0.8 g | |
| | | 200.0 g | (10.0 g as solid) |

[* : Fluorescein Sodium solution used in the above formulation is a ethanolic (100 ml) solution containing Fluorescein Sodium (30 mg)]

<u>Example 3</u>

(1) The bed granules prepared in accordance with Example 1-(1) was coated in a convention a manner with the coating solution prepared in accordance with Example 2-(1) using a fluid bed granulator to form the microcapsules. Four different amounts of the coating materials in terms of percentage** were used as follows;

6%, 8%, 10%, 15% (Eudragit E 30 D formulation)

(**: The percentage of the coating material comes from the ratio of the amount of the coating material to that of the bed granules on the assumption that all coating solutions sprayed were adsorbed to the granules.

(2) The bed granules prepared in accordance with Example 1-(1) was coated in a convention a manner with the coating solution prepared in accordance with Example 2-(4) using a fluid bed granulator to form the microcapsules. Four different amounts of the coating materials in terms of percentage** were used as follows;

3.5%, 5%, 10%, 15% (Ethylcellulose formulation)

(3) The bed granules prepared in accordance with Example 1-(1) was coated in a convention a manner with the coating solution prepared in accordance with Example 2-(2) using a fluid bed granulator to form the microcapsules. Four different amounts of the coating materials in terms of percentage** were used as follows;

6% (Eudragit E 30 D formulation)

(4) The bed granules prepared in accordance with Example 1-(2) was coated in a convention a manner with the coating solution prepared in accordance with Example 2-(3) using a fluid bed granulator to form the microcapsules. Four different amounts of the coating materials in terms of percentage** were used as follows;

4%, 6%, 8%, 10%, 12%, 15% (Eudragit E 30 D formulation)

(5) The bed granules prepared in accordance with Example 1-(2) was coated in a convention a manner with the coating solution prepared in accordance with Example 2-(5) using a fluid bed granulator to form the microcapsules. Four different amounts of the coating materials in terms of percentage** were used as follows;

4%, 6%, 8%, 10%, (Ethylcellulose formulation)

(6) The bed granules prepared in accordance with Example 1-(3) was coated in a convention a manner with the coating solution prepared in accordance with Example 2-(6) using a

fluid bed granulator to form the microcapsules. Four different amounts of the coating materials in terms of percentage** were used as follows;

4%, 6%, 8%, 10%, (Ethylcellulose formulation)

(7) The bed granules prepared in accordance with Example 1-(3) was coated in a convention a manner with the coating solution prepared in accordance with Example 2-(6) using a fluid bed granulator to form the microcapsules. Four different amounts of the coating materials in terms of percentage** were used as follows;

4%, 6%, 8%, 10%, (Ethylcellulose formulation)

According to the present invention, superior sustained-release microcapsule can be obtained by using Eudragit or ethylcellulose as the release controlled membrane of the drug, in which the release rate of the drug is scarcely influenced by the change of the stirring intensity and the pH value, and the drug is released in established zero order release.

In the present invention, by adjusting the amount of the coating of the release controlled membrane of the drug, the release rate of the drug from microcapsules can be controlled sustainedly.

Namely, the rate-determining step of the drug release from the microcapsule of the present invention is the step where the release of the drug from the coating membrane, and this characteristic gives rise to the

feature of the sustained-release preparations of the present invention that the dissolution pattern of the drug is linear and that the dissolution pattern and the dissolution rate scarcely get influenced by the change of the intensity of stirring, the pH of the aqueous medium, or the like.

In the following, in order to illustrate the effect obtained by the present invention, the representative test results are given.

Test preparations

A : The microcapsule described hereinbefore in Example 3-(1) (the amount of the coating material: 6%) (Eudragit E30D formulation)

B : The microcapsule described hereinbefore in Example 3-(1) (the amount of the coating material: 8%) (Eudragit E30D formulation)

C : The microcapsule described hereinbefore in Example 3-(1) (the amount of the coating material: 10%) (Eudragit E30D formulation)

D : The microcapsule described hereinbefore in Example 3-(1) (the amount of the coating material: 15%) (Eudragit E30D formulation)

E : The microcapsule described hereinbefore in Example 3-(2) ( the amount of the coating material: 3.5%) (Ethylcellulose formulation)

F : The microcapsule described hereinbefore in Example 3-(2) (the amount of the coating material: 5%) (Ethylcellulose formulation)

G : The microcapsule described hereinbefore in Example 3-(2) (the amount of the coating material: 10%) (Ethylcellulose formulation)

H : The microcapsule described hereinbefore in Example 3-(2) (the amount of the coating material: 15%) (Ethylcellulose formulation)

Dissolution tests (Paddle method)

Test methods

(1) The dissolution test disclosed in the pharmacopoeia of Japan (10th eddition) (2nd method) (distilled water, 100 rpm, 900 ml)

(2) The dissolution test disclosed in the pharmacopoeia of Japan (10th eddition) (2nd method) (distilled water, 50 rpm, 900 ml)

(3) The dissolution test disclosed in the pharmacopoeia of Japan (10th eddition) (2nd method) (distilled water, 200 rpm, 900 ml)

(4) The dissolution test disclosed in the pharmacopoeia of Japan (10th eddition) (2nd method) (1st fluid, 100 rpm, 900 ml)

(5) The dissolution test disclosed in the pharmacopoeia of Japan (10th eddition) (2nd method) (2nd fluid, 100 rpm, 900 ml)

Each sample contains 30 mg as 2-nitroxymethyl-6-chloropyridine.

All of the tests were performed at 37°C (±0.5°C) and quantitative analyses were performed in UV method (268 nm).

To investigate the influence of the difference in conditions of the dissolution test, i.e., intensity of stirring and pH of test solution, on the dissolution pattern, a series of tests were carried out under several conditions shown in Table 2 - 5.

Table 2. Dissolution (%) at each time

| Test method | Test preparation | Time (hr) | | | |
|---|---|---|---|---|---|
| | | 2 | 4 | 6 | 8 |
| (1) | A | 15 | 36 | 54 | 67 |
| | B | 12 | 26 | 40 | 55 |
| | C | 7 | 20 | 31 | 44 |
| | D | 4 | 11 | 19 | 28 |

Table 3. Dissolution (%) at each time

| Test method | Test preparation | Time (hr) | | | |
|---|---|---|---|---|---|
| | | 2 | 4 | 6 | 8 |
| (1) | E | 43 | 68 | 82 | 89 |
| | F | 18 | 34 | 44 | 56 |
| | G | 8 | 15 | 22 | 27 |
| | H | 4 | 9 | 13 | 17 |

Table 4. Dissolution (%) at each time

| Test preparation | Test method | Time (hr) | | | |
|---|---|---|---|---|---|
| | | 2 | 4 | 6 | 8 |
| A | (2) | 15 | 35 | 56 | 67 |
| | (1) | 15 | 36 | 54 | 67 |
| | (3) | 17 | 33 | 54 | 68 |

Table 5. Dissolution (%) at each time

| Test Preparation | Test method | Time (hr) | | | |
|---|---|---|---|---|---|
| | | 2 | 4 | 6 | 8 |
| A | (1) | 15 | 36 | 54 | 67 |
| | (4) | 15 | 29 | 44 | 59 |
| | (5) | 15 | 31 | 48 | 63 |

From the dissolution test results given in Tables2 and 3, it turned out that in either cases that Eudragit and ethylcellulose were used as the coating material capable of controlling the release of the drug, 2-nitroxymethyl-6-chloropyridine was dissolved at zero order release, and said release rate became late in propotion to the amount of the coating. Therefore, by controlling the amount of the coating the desired release rate can be selected.

Further, from the result given in Table 4, it turned out that the sustained-release preparations of the present invention had the characteristic that even if the intensity of stirring was changed, the dissolution pattern was almost linear (namely, the dissolution rate was nearly constant) and further the dissolution rate was scarcely influenced by the changes of the dissolution conditions.

Furthermore, from the result given in Table 5, it became clear that both the dissolution pattern and the dissolution rate were scarcely influenced by the change of the pH value of the dissolution medium.

What we claim is :

1. A sustained-release pharmaceutical preparation which comprises 2-nitroxymethyl-6-chloropyridine or an occlusion compound thereof with β-cyclodextrin.

2. A sustained-release preparation of claim 1, which comprises an occlusion compound of 2-nitroxymethyl-6-chloropyride with β-cyclodextrin.

3. A sustained-release preparation of claim 1, wherein pharmaceutical form is microcapsule.

4. A sustained-release preparation according to any of claims 1 to 3, characterized by the fact that granules containing the active compound are coated with released control membrane.

5. Process for preparing a sustained-release preparation comprising 2-nitroxymethyl-6-chloropyridine or an occlusion compound thereof, characterized by the fact, that granules containing 2-nitroxymethyl-6-chloropyridine or an occlusion compound thereof are coated with released control membrane.

6. Process according to claim 5, characterized by the fact, that microcapsules are prepared.

7. Use of the preparation of claim 1 as a medicament.

8. Use of the prepartion of claim 1 in the treatment of vascular disorder.